# EUROPEAN PATENT SPECIFICATION

(11) **EP 2 051 717 B1**
(45) Date of publication and mention of the grant of the patent: **13.07.2011**
(21) Application number: 07734535.3
(22) Date of filing: 08.05.2007
(51) Int. Cl.: A61K 33/34, A61P 9/00

(54) **REPLACING HEART SURGERY BY MICRONUTRIENT**
ERSATZ EINER HERZOPERATION DURCH MIKRONÄHRSTOFF
CHIRURGIE CARDIAQUE REMPLACEE PAR DES MICRONUTRIMENTS

(30) Priority: 14.08.2006 GB 0616201
(43) Date of publication of application: 29.04.2009
(73) Proprietor: SHAHNAZ, Bibi, Kohat (PK)
(72) Inventor: AZMAT, Hayat, Kohat (PK); BIBI, Mamoona, Kohat (PK); SHAHNAZ, Bibi, Kohat (PK)
(74) Representative: Dörries, Hans Ulrich
(86) International application number: PCT/IB2007/001225
(87) International publication number: WO 2008/020275

(56) References cited:
- WO-A-00/67781
- DE-A1- 10 020 674
- MISRA R ET AL: "Serum copper, ceruloplasmin & iron in ischaemic heart disease" INDIAN HEART JOURNAL, CARDIOLOGICAL SOCIETY OF INDIA, CALCUTTA,, IN, vol. 30, no. 6, November 1978 (1978-11), pages 339-344, XP009089030 ISSN: 0019-4832
- ATANASIU R ET AL: "Class III antiarrhythmic effects of ceruloplasmin on rat heart" CANADIAN JOURNAL OF PHYSIOLOGY AND PHARMACOLOGY, OTTAWA, ONT, CA, vol. 74, 1996, pages 652-656, XP002144158
- ATANASIU ROXANA ET AL: "Antiarrhythmic effects of ceruloplasmin during reperfusion in the ischemic isolated rat heart" CANADIAN JOURNAL OF PHYSIOLOGY AND PHARMACOLOGY, OTTAWA, ONT, CA, vol. 73, no. 9, 1995, pages 1253-1261, XP009089048

## Description

The invention is related with the heart cure by micronutrients/trace elements without open-heart surgery.

According to Paracelsus, the Chief iatro chemist (The prefix iatro- represents the Greek iatros, "physician") of the early sixteenth century, "the object of chemistry is not to make gold but to prepare medicines". Paracelsus regarded the human body as simply a combination of chemicals and chemical processes. Illness, he declared, was caused by certain chemical processes going astray; consequently illness could be cured only by chemical medicines Paracelsus theory was based upon one first suggested, supposedly, by Basilius Valentinus held that all substances were composed of mercury, sulfur and salt when these are not present in right proportion in the body, said he, illness results. Thus, by the proper intake of one or another ingredient, balance could again be restored (1).

THE CURRENT INVENTION IS THE PRACTICAL PROOF OF PARACELSUS ABOVE SAID THEORY.
An essential nutrient is one for which a deficiency results in impairment of function that is relieved only by administration of that substance. Essentiality is organism-dependent, and care must be taken to distinguish it from stimulation. There are many instances of both essential and nonessential compounds behaving as stimulants. Some substances at some concentrations may be merely inert or innocuous, without evident effect. Inert metals such as tantalum, platinum, silver, and gold are often employed as surgical implants. There are numerous compounds that serve as therapeutic agents. Examples include the historical use of arsenic and mercury compounds against parasites and the employment of lithium in manic depression (2).

Heart is a busy pump linked by 100,000 miles of pipelines to all parts of body and its pipelines ensure the continuous flow of oxygen rich blood to brain, lungs, kidneys and other vital organs need. The weight of heart in an average person is less than one pound and is as big as the fist. It beats about 70 times a minute and more than 100,000 times in a single day .It pumps 4.7 liters of blood through its chambers every 60 seconds. It works in one hour to lift a weight of 1.4 metric tons (3).

Diseases of the heart and blood vessels are called cardiovascular diseases. The three most important kinds of heart diseases are, (a) arteriosclerosis or hardening of the arteries, (b) hypertension or high blood pressure, (c) rheumatic fever, which develops rheumatic heart disease later in life.

Heart attacks are the greatest single cause of death in United States. Every day they kill 1,800 persons and leave thousands of others crippled to some extent for the rest of their lives (4-5).

A heart attack occurs suddenly, but the factors that cause it take many years to build up (6).

Already existing treatment of heart problems are, repairing holes in the heart, repairing a damaged heart by open-heart surgery, treating coronary artery disease by drugs or surgery. Abnormal heart rhythms are corrected by drugs. Heart failure can be avoided by assisting heart by drugs to pump blood (7-11).

The existing methods for the cure does not eradicate the heart diseases, are tedious, expensive, time consuming, involve life risk and require high technicality. Mainly in allopathic the method of treatment is based on surgery at the site of problem accompanied by the use of so many drugs life long in large quantities. These drugs usually have deleterious effects on human health and the long time use of these drugs usually results in the development of more diseases (usually called side effects of the medicine). Moreover, surgery is a risky job and needs well-equipped operation theaters.

The current invention is,
a. Easily carried out
b. Ever cheapest technology
c. Gives 100% cure
d. No use of lifelong drugs
e. Accompanied by no risk of life
f. No side effects
g. Nature friendly
h. Does not need most expensive and complicated equipment
i. Needs no hospitalization
j. No need of restrictions on taking the cholesterol and fat diet after cure if followed the diet schedule
k. No need of extra exercise except necessary for carrying out the work for daily life.

The current technology is based on the use of copper sulphate. In particular, the invention relates to a solution of the micronutrient copper sulphate for use in opening the blockage or narrowing of blood vessels in the treatment or prevention of heart diseases by producing ceruloplasmin. Preferably, the opening of the blockage or narrowing of blood vessels cures or prevents heart disease, except holes and low blood pressure, of a patient or the opening of the blockage or narrowing of blood vessels cures or prevents high blood pressure of a patient. The present invention also relates to a solution of the micronutrient copper sulphate for use in curing brain hemorrhage of a patient by producing ceruloplasmin.

Medicine can be made by dissolving of ferrous sulphate.(1)= ten mg in five ml of boiled concentrated sugar cane juice, manganese sulphate(2)= twenty mg in ten ml of boiled concentrated sugar cane juice, copper sulphate(3)= five hundred mg in ten ml of concentrated boiled sugarcane juice, all the three solutions thus prepared are transferred to 100 ml volumetric flask and the volume is raised with boiled concentrated sugar cane juice up to the mark of volumetric flask. From conical flask, five ml sterilized droppers are filled and one to three drops per day are given to the heart patients. Doses of the said medicine are at level of parts per billion and parts per million) on fasting in morning at least one hour before breakfast. The patients in serious conditions should be given minimum doses, i.e., one or two drops along with the use of routine allopathic medicines (usually with a gap of one to three hours in the use of this medicine and allopathic medicines), which are gradually reduced with the improvement in the condition of patient. The infants should be given one drop at the time when the milk has been digested and no milk should be given at least half an hour after giving the medicine. No allopathic medicines should be given except for the life risks and if given, should not be given close to the invented medicine as the medicine is wasted by the use of allopathic medicines. The approaches of two technologies, i.e., current technology and allopathic, are almost opposite to each other. So, no allopathic medicine should be given to patients after giving the medicine if fever, flue, vomiting, motions and pain appear anywhere in the body (which will result ultimately in the loss of weight and disappearance in swelling on the body) as these symptoms are the part of cure and should not be treated with allopathic medicines. These symptoms show that the medicine is working at the point of problem and if tried to kill the system (by allopathic ordinary medicines like aspirin which is very easily done) developed by the medicine, whole of the work done by the medicine in the body of the patients will be wasted and the patient will have to go through the same conditions if again brought on the invented medicine. The disappearance time of these symptoms will depend on the body of patients whether they have taken the natural diet or industrial diet or the food without fertilizers or the food of fertilizers or on the extent of using allopathic medicine. The patients having natural diet and no use of allopathic medicines will be recovered soon while others may take greater time. Some patients may take comparatively greater time for cure if they use drinking water with higher calcium content. So, it is preferable for patients to boil drinking water to separate calcium bicarbonate and then use water for drinking purposes. Sometimes, patients do complaint of weakness, it is because that the body system becomes alive and starts to detect the problems of the body. Flue is common in this method of curing and should not be stopped by using allopathic drugs. After the cure, the patients become active and feel no problem while going upstairs. The medicine works well if carbohydrates, protein rich diet and small amount of table salt in diet is given. During treatment the blood pressure may rise and the cholesterol levels in the blood may go higher but the allopathic medicines for the control of both symptoms should be avoided. For both of the symptoms, i.e., high blood pressure and high cholesterol levels in the blood should be controlled by two ways,
1. By lowering the dose of the medicine, i.e. from three drops to one drop per day,
2. By using the allopathic medicines for excessive urination.

In the cases where the condition of patients is serious, the doses of the medicine should be the lowest, i.e., one drop per day and when the condition becomes stable, slowly and gradually the doses are increased.

The medicine itself diagnoses and cures. No need of diagnoses first in majority of cases, the only caution is to start with lower doses, i.e., one to three drops per day which can be repeated in a day with improvement in the condition of patient. The body of a patient will talk in the form of pain or relief (which indicate the point of problem) and the one has to follow the track in which direction one will guide the body of a patient by administering the required micronutrients. The functioning of the system developed by micronutrients is that the symptoms appearing in any of the diseases, may go higher by micronutrients first, like, in case of fever of 100°C, may go to 104°C, no other medicine is required, only sponging etc, wait for some days with the same doses of micronutrients, slowly the temperature will start to go downward or fluctuation may occur and then finishes altogether. Sometimes, some symptoms of diseases may appear which were not at the time of giving micronutrients, the reason for this is that in majority of cases, the patient's bodies have deep seated germs which are checked and cured by the body mechanism developed by micronutrients. So, it is possible that if micronutrients given at the age of eighties may check the disease of his childhood after eighty years, which the patient has not, mentioned all together at the start of taking medicine. So, taking history of patient, each and every disease in life should be taken into account although it may have not appeared again in life but the chances are there for the checking. Same is the case of motions, vomiting etc which may not be present at the time of giving micronutrient but the patient may have the history of motions, so, the motions may appear but no need of controlling them, only administer the dehydration or mineral deficiency accompanied by excessive motions. They will appear with the same medicine and will finish with the same medicine.

For further details, please visit the web site, www.latestimmunopathy.com

### EXPERIMENTAL

The recent study is related with the repairing and curing of heart by medicine, i.e., enlarge heart, curing coronary artery disease or triple vessel disease and other related diseases except holes. 5-ml. blood is extracted according to international standards for trace elements, keep at 4°C and let to clot for the separation of serum. Electrophoresis of the serum is carried out to separate the different protein groups and is analyzed by atomic absorption instrument for metal concentrations.

### CAUTION

1. This medicine is strictly prohibited for patients having transplants in their bodies like spring, valves etc., except the patients having lenses in their eyes, where one drop can be given.
2. Medicine works better if boiled drinking water is used and more proteins and carbohydrates are used.
3. During treatment, following items are strictly prohibited to take in meals (food prepared by involvement of industry is prohibited like fortified flour etc).
   a. Milk and milk products
   b. Cooking oil {lifelong}
   c. Dry fruit
   d. Fish
   e. Pulses
   f. Farm chicken and farm eggs (life long)
   g. Iodized table salt without knowing the deficiency h. Food from fortified flour {lifelong}
   i. Food prepared by industry {lifelong}

### HOW THE MEDICINE WORKS

When an electrolyte is dissolved to make a solution, it is dissociated into positive and negative ions. In the same way, micronutrient copper sulphate is also dissociated into positive ion copper and negative ion sulphate.

The positive ion is absorbed in the alimentary canal and picked up by a protein called albumin present in the blood and transported to the liver. Liver synthesizes the most important molecule called ceruloplasmin (Ceruloplasmin being part of immune system).

When the molecule has been synthesized, it is released into the blood stream. During circulation in the blood stream, it reacts with the calcium carbonate settled along with cholesterol within the walls of blood vessels. So, in this way, the settled material within the walls of blood vessels slowly starts to dissolve setting free the settled cholesterol and the salt, "calcium carbonate" after reacting with the molecule ceruloplasmin and resulting in the formation of copper carbonate.

If the rate of dissolution is higher than the rate of excretion from the kidney, then the blood pressure starts to elevate from the normal limit. If this stage comes, then either the dose of medicine is reduced or allopathic medicine for urination is given. The schematic reaction taking place after the formation of the molecule is shown in annexure A (total lines=twenty).

The formation and concentration of the ceruloplasmin molecule in blood is dependent upon two factors,
i. Higher concentrations of calcium in the blood because calcium in the blood decomposes the molecule present in the blood before its function on the blockage in the heart or anywhere blockage within the body.
ii. Higher concentration of vitamin-E retards the formation of the molecule ceruloplasmin which is only and only functional molecule against the heart disease.

1. As a result of medicines given to patient, ceruloplasmin molecule is produced within the body of heart patients. This molecule is multifunctional in nature and performs according to the situation faced by the body of patients. In this particular situation of heart diseases, this molecule is capable of opening the blockage or narrowing of vessels, valves etc, by the reaction shown in Annex-1.

### Annexure A (Page-1)

Calcium and Copper are in the same period of Periodic Table. But Calcium is in the second Group of Periodic Table having two electrons in the outermost Shell. Copper is a transition metal in the same Period of Calcium and reactivity of elements increases along a period. So, Copper is more reactive as compared with the calcium. When Copper Sulphate solution is made, it dissociates into Copper ion, Cu⁺⁺ and SO⁻4. Copper ion is absorbed in the alimentary canal and picked up by albumin protein of blood plasma and transported to Liver where Ceruloplasmin is synthesized. Ceruloplasmin has eight atoms of copper attached with it. When Ceruloplasmin circulates in blood, it attacks calcium carbonate settled within the walls of blood vessels or blocked vessels and then settled calcium carbonate is dissociated to form copper carbonate and in this way settled cholesterol is freed and begins to circulated within the blood. In this way blockage of vessel goes away.

Schematic reaction is shown on next page. 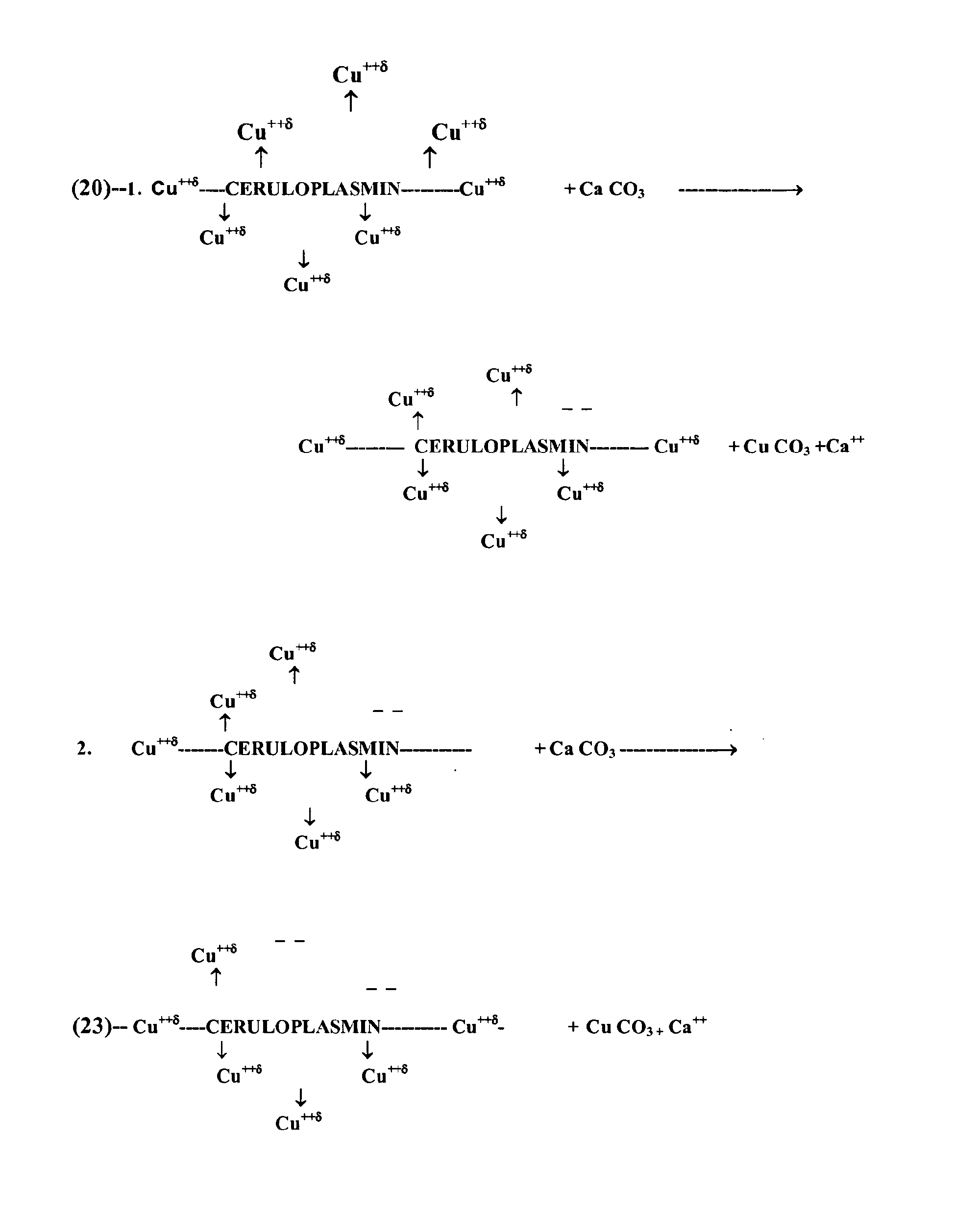
2. The molecule ceruloplasmin is also capable to cure the heart damage after heart attack but the doses should be very low after heart attack along with the use of parallel use of allopathic medicine but maximum gap should be kept between the invented medicine and allopathic medicine.
3. The molecule ceruloplasmin is capable of repairing the body at cellular level and mutations are cured in the presence of ceruloplasmin molecule. So, there is no genetic disease.
4. The molecule ceruloplasmin is capable of checking the cancer cells at cellular level and during checking the patients will fell needle like pain at the place of cancer cell within the body.
5. The molecule ceruloplasmin function is affected in the presence of excess calcium and excess Vitamin-E. As Vitamin-E retards the metabolic rate, so in the presence of excess amount of Vitaman-E, there will be lesser production of the molecule ceruloplasmin. So excess of Vitamin-E is the door to death as the lesser number of the molecule ceruloplasmin means lesser defense and that the body of patient will be open for attack of every kind of disease.
6. Excess calcium within the blood will decompose the molecule ceruloplasmin before performing functions for care or maintenance of health. So, excess calcium is 2^{nd} door to death.
7. The micronutrient copper suphate dissolved in 100ml of sterilized water will work in the same way as the above medicine.
8. The micronutrient copper sulphate dissolved in 100ml with combination of trace amounts of all trace metals existing on earth including toxic metals dissolved in the same above 100 ml solution will work effectively against the disease provided the un-required metal concentration is kept below the concentration of invented medicine.

NOTE:- This technology is only for heart patients having blockade in heart (or anywhere in the body) and high blood pressure only and not for any additional disease like diabetes etc., i.e., it is not applicable for heart patients having diabetes, pains in joints etc.

### THE MOLECULE CERULOPLASMIN HOLDS TOP MOST POSITION IN IMMUNE SYSTEM AND IS CAPABLE OF DIAGNOSING THE SITE OF PROBLEM AND THEN REPAIRING IT.

### REFERENCES

1. John H. Secrist and Wendell H. Powers, Prof. Of Chemistry - Wayne State Univ., Canada, General Chemistry Page - 19-20, 1966.
2. H. Sigel (ed.),"Inorganic Drugs in Deficiency and Disease," Vol. 14 of Metal Ions in Biological Systems, Marcel Dekker, New York, 1982.
3. The World Book Encyclopedia World Book-Child craft International Inc. Vol. 9, P.132, 1980, USA.
4. The World Book Encyclopedia 9 (137-138) 1980, USA.
5. The World Book Encyclopedia 9(138) 1980, USA.
6. The World Book Encyclopedia 9(139) 1980, USA.
7. The World Book Encyclopedia 9(140-140a) 1980, USA.
8. Keno, Hikaru (Sch. Med. Kyshu Uni. Fukuoka, Japan, 812-821, Saishin Igaku 1997, 52(10), (2309-2317) Japan.
9. Liu, Yao, Guo, Quliang; Caj, Hongwei; Huang, Qingin (Department of Anesthesis, Human Medical University Affiliated Xiangin Hospital, Chansha, Peop. Rep. China, 410008, Human Yike Danuae Ziabao 1998, 23(2), 152-154(Ch).
10. Clesphas, Ton J.M.; Niemeyer, Menlo G; Bermink, Piet J. Z.M.; Zwinderman, Koos H; Wijk, Anton V; Wall, Ernst E.V.D. (Department Medicine Merwede Hospital Dordrecht, 3360 AB Sliedrecht, Neth.) Curr. Ther. Res. 57(8), 614-621, 1996. (Eng).
11. Soerenson, Jens Ahm, Anderson Ole (Dep. Environm., Med., Odense Univ. Odense, DK-5000 Den). Pharmacist Toxic of (Copenhagen) 65(3) 209-13, 1989 (Eng.).

**TABLE No.I**

| **NORMAL FEMALE SUBJECTS IN MARDAN CITY OF PAKISTAN** | | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 35-50 | Meat, Fruit &Vegetable | Municipal Source | 550-800 | 700 |
| 2 | 50 | 30-50 | Pulses & vegetables | Municipal Source | 400-450 | 430 |
| 3 | 50 | 30-50 | Meat, Fruit &Vegetable | Local Well | 420-500 | 450 |
| 4 | 50 | 30-50 | Meat, Fruit &Vegetable | Local well other than above | 400-550 | 500 |

**TABLE No. II**

| **FEMALE HEART PATIENTS USING COOKING OIL IN MARDAN CITY OF PAKISTAN** | | | | | | |
|---|---|---|---|---|---|---|
| **S. No.** | **No. OF PATIENTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 35-50 | Meat, Fruit &Vegetable | Municipal Source | 400-500 | 450 |
| 2 | 50 | 30-50 | Pulses & vegetables | Municipal Source | 400-450 | 430 |
| 3 | 50 | 30-50 | Meat, Fruit &Vegetable | Local Well | 420-500 | 450 |
| 4 | 50 | 30-50 | Meat, Fruit &Vegetable | Local well other than above | 400-550 | 500 |

**TABLE No. III**

| **NORMAL MALE SUBJECTS WITHOUT USING COOKING OIL AND IODISED TABLE SALT IN MARDAN CITY OF PAKISTAN** | | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 45-60 | Meat, Fruit &Vegetable | Municipal Source | 800-900 | 850 |
| 2 | 50 | 45-60 | Pulses & vegetables | Municipal Source | 600-700 | 650 |
| 3 | 50 | 45-60 | Meat, Fruit &Vegetable | Local Well | 820-900 | 850 |
| 4 | 50 | 45-60 | Meat, Fruit &Vegetable | Local well other than above | 600-750 | 700 |

**TABLE No. IV**

| **NORMAL MALE SUBJECTS USING COOKING OIL IN MARDAN CITY OF PAKISTAN** | | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 35-50 | Meat, Fruit &Vegetable | Municipal Source | 600-750 | 650 |
| 2 | 50 | 35-50 | Pulses & vegetables | Municipal Source | 500-600 | 520 |
| 3 | 50 | 35-50 | Meat, Fruit &Vegetable | Local Well | 600-650 | 620 |
| 4 | 50 | 35-50 | Meat, Fruit &Vegetable | Local well other than above | 650-750 | 700 |

**TABLE No. V**

| **MALE HEART PATIENTS USING COOKING OIL IN MARDAN CITY OF PAKISTAN** | | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 45-60 | Meat, Fruit &Vegetable | Municipal Source | 500-650 | 550 |
| 2 | 50 | 45-60 | Pulses & vegetables | Municipal Source | 450-500 | 480 |
| 3 | 50 | 45-60 | Meat, Fruit &Vegetable | Local Well | 500-600 | 550 |
| 4 | 50 | 45-60 | Meat, Fruit &Vegetable | Local well other than above | 500-620 | 550 |

**TABLE No. VI**

| **NORMAL FEMALE SUBJECTS WITHOUT USING COOKING OIL AND IODISED TABLE SALT IN KOHAT CITY OF PAKISTAN** | | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 35-50 | Meat, Fruit &Vegetable | Municipal Source | 850-1000 | 900 |
| 2 | 50 | 30-50 | Pulses & vegetables | Municipal Source | 750-900 | 800 |
| 3 | 50 | 30-50 | Meat, Fruit &Vegetable | Local Well | 900-950 | 920 |
| 4 | 50 | 30-50 | Meat, Fruit &Vegetable | Local well other than above | 700-850 | 800 |

**TABLE No. VII**

| | **NORMAL FEMALE SUBJECTS USING COOKING OIL IN KOHAT CITY OF PAKISTAN** | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 35-50 | Meat, Fruit &Vegetable | Municipal Source | 400-500 | 450 |
| 2 | 50 | 30-50 | Pulses & vegetables | Municipal Source | 400-450 | 420 |
| 3 | 50 | 30-50 | Meat, Fruit &Vegetable | Local Well | 450-500 | 420 |
| 4 | 50 | 30-50 | Meat, Fruit &Vegetable | Local well other than above | 400-550 | 500 |

**TABLE No. VIII**

| **FEMALE HEART PATIENTS USING COOKING OIL IN KOHAT CITY OF PAKISTAN** | | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 35-50 | Meat, Fruit &Vegetable | Municipal Source | 400-500 | 430 |
| 2 | 50 | 30-50 | Pulses & vegetables | Municipal Source | 400-450 | 420 |
| 3 | 50 | 30-50 | Meat, Fruit &Vegetable | Local Well | 400-550 | 450 |
| 4 | 50 | 30-50 | Meat, Fruit &Vegetable | Local well other than above | 400-500 | 450 |

**TABLE No. IX**

| **NORMAL MALE SUBJECTS WITHOUT USING COOKING OIL AND IODISED TABLE SALT IN SWAT VELLY OF PAKISTAN** | | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 35-45 | fish | Local Well | 600-800 | 650 |
| 2 | 50 | 35-45 | Fish & pulses | Municipal Source | 650-700 | 620 |
| 3 | 50 | 35-45 | Fish & pulses | Local Well | 600-750 | 650 |
| 4 | 50 | 35-45 | Fish & pulses | Local well other than above | 500-700 | 600 |

**TABLE No. X**

| **NORMAL MALE SUBJECTS WITHOUT USING COOKING OIL AND IODISED TABLE SALT IN SWAT VELLY OF PAKISTAN** | | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 35-45 | fish | Spring water | 600-750 | 650 |
| 2 | 50 | 35-45 | Fish & pulses | Spring water | 550-700 | 600 |
| 3 | 50 | 35-45 | Fish & pulses | Spring water | 500-650 | 550 |
| 4 | 50 | 35-45 | Fish & pulses | Spring water | 500-700 | 600 |

**TABLE No. XI**

| **NORMAL MALE SUBJECTS USING COOKING OIL IN SWAT VELLY OF PAKISTAN** | | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 35-45 | Meat, Fruit &Vegetable | Municipal Source | 650-750 | 700 |
| 2 | 50 | 30-45 | Pulses milk &vegetables | Municipal Source | 500-600 | 550 |
| 3 | 50 | 30-45 | Meat, Fruit & Vegetable | Local Well | 600-750 | 650 |
| 4 | 50 | 30-45 | Meat, Fruit &Vegetable | Local well other than above | 650-750 | 700 |

**TABLE No. XII**

| **MALE HEART PATIENTS USING COOKING OIL IN SWAT VELLY OF PAKISTAN** | | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 35-65 | Meat, Fruit &Vegetable | Local Well | 600-750 | 680 |
| 2 | 50 | 35-65 | Pulses, milk &vegetables | Local Well | 500-600 | 520 |
| 3 | 50 | 35-65 | Eggs, Fruit &Vegetable | Local Well | 600-650 | 620 |
| 4 | 50 | 35-65 | Pulses, milk &Vegetable | Local Well | 500-650 | 600 |

**TABLE No. XIII**

| **MALE HEART PATIENTS WITHOUT USING COOKING OIL IN SWAT VELLY OF PAKISTAN** | | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 35-65 | Meat, Fruit &Vegetable | Local Well | 800-950 | 900 |
| 2 | 50 | 35-65 | Pulses, milk &vegetables | Municipal source | 700-800 | 750 |
| 3 | 50 | 35-65 | Eggs, Fruit &Vegetable | Municipal source | 800-850 | 820 |
| 4 | 50 | 35-65 | Pulses, milk &Vegetable | Municipal source | 600-700 | 650 |

**TABLE No. XIV**

| **NORMAL MALE SUBJECTS USING COOKING OIL IN PESHAWAR CITY OF PAKISTAN** | | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 35-45 | Meat, &Vegetable | Municipal Source | 600-700 | 650 |
| 2 | 50 | 30-45 | Pulses, milk &vegetables | Municipal Source | 600-650 | 620 |
| 3 | 50 | 30-45 | Milk, pulses | Municipal Source | 550-650 | 600 |
| 4 | 50 | 30-45 | Meat, Fruit &Vegetable | Municipal Source | 600-650 | 630 |

**TABLE No. XV**

| **NORMAL MALE SUBJECTS USING IODISED TABLE SALT IN PESHAWAR CITY OF PAKISTAN** | | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMM (ppb)** | **MEDIAN** |
| 1 | 50 | 35-55 | Meat, pulses&Vegetable | Municipal Source | 600-700 | 650 |
| 2 | 50 | 30-55 | Pulses, milk &vegetables | Municipal Source | 550-600 | 580 |
| 3 | 50 | 30-55 | Milk, pulses | Municipal Source | 500-600 | 550 |
| 4 | 50 | 30-55 | Meat, Fruit &Vegetable | Municipal Source | 550-750 | 600 |

**TABLE No. XVI**

| **MALE HEART PATIENTS USING COOKING OIL IN PESHAWAR CITY OF PAKISTAN** | | | | | | |
|---|---|---|---|---|---|---|
| **S. No.** | **No. OF SUBJECTS** | **AGE IN YEARS** | **EATING HABITS** | **TYPE OF DRINKING WATER** | **CONCENTRATION OF COPPER IN CERULOPLASMIN (ppb)** | **MEDIAN** |
| 1 | 50 | 35-55 | Meat, pulses&Vegetable | Municipal Source | 500-550 | 520 |
| 2 | 50 | 30-55 | Pulses, milk &vegetables | Municipal Source | 550-580 | 570 |
| 3 | 50 | 30-55 | Milk, pulses | Municipal Source | 500-580 | 530 |
| 4 | 50 | 30-55 | Meat, Fruit &Vegetable | Municipal Source | 500-600 | 550 |

### DISCUSSION OF TABLES

### TABLE No. I

Table No. 1 shows the concentration of copper in normal female subjects of Mardan city. The age limit is the same between different groups of different eating habits with different source of drinking water. The group in serial No. 1 has the highest concentration of copper due to the meat and fruit in food as compared with the second group. In serial No. 2, the concentration of copper is low as compared with the group in serial No. 1 with the same source of drinking water; the reason for the difference in concentration is due to the difference in eating habits. Pulses in the food of group in serial No. 2, lowers the formation of ceruloplasmin which lowers the concentration of copper. While the lower concentration of ceruloplasmin in groups of serial No. three and four is due to the difference in the source of drinking water which decomposes the molecule of ceruloplasmin due to the higher calcium levels in drinking water. So, the groups in serial No. 2, 3 and 4 are more prone toward the heart disease as compared with the group in serial No. 1.

### TABLE No. II

Table No. 2 shows the concentration of ceruloplasmin in different groups of heart patients. On looking carefully on the table, it appears that the reason for low concentration of copper in all serial groups is the use of cooking oil irrespective of the drinking water source, which is responsible for the onset of heart disease.

### TABLE No. III

Table No. 3 shows the concentration of copper in normal male subjects without using cooking oil and iodized table salt in Mardan city of Pakistan.

On comparison of the groups in serial No. 1 and 2, it is evident that low concentration of copper in group of serial No. 2 is the use of pulses in their food, which contain vitamin E responsible for lowering the concentration of copper (vitamin E slows down the metabolic rate and reducs the production of the molecule ceruloplasmin). So, the group in serial No. 2 is more prone for heart diseases as compared to group in serial No. 1. On comparison of groups in serial No. 3 and 4, eating habits are the same but there is a difference in calcium concentration (of drinking water. The reason for the low concentration of copper in the group of serial No. 4 is drinking water with high concentration of calcium which decomposes the molecule of ceruloplasmin and thus lowering the immune system, which ultimately increases the risk of heart diseases.

### TABLE No. IV

The table shows the concentration of copper in the molecule of ceruloplasmin in normal male subjects using cooking oil in Mardan city of Pakistan. On comparison of groups in serial No. 1 and 2, water source is the same, so the difference in the concentration is due to the eating habits of the groups. The group in serial No. 2, eat pulses which contain maximum concentration of (435) vitamin E which retards the metabolic process resulting in the low concentration of the molecule ceruloplasmin. So, the group in serial No. 2 is more prone to heart diseases as compared to group from serial No. 1. Looking at the concentration of copper in the groups of serial No. 3 and 4, the eating habits are the same which normally would have higher concentration of copper in the molecule of ceruloplasmin but the result shows lower concentrations, so, it appears that the drinking water in both of the groups has higher concentrations of calcium which decomposes the molecule of ceruloplasmin in immune system. So, the groups in serial No. 3 and 4 will also be more prone to heart diseases.

### TABLE No. V

The table shows the record of male heart patients using cooking oil in Mardan city of Pakistan. The concentration of copper in the molecule of ceruloplasmin is low in each group irrespective of their eating habits. The reason for lower values of copper in the molecule of ceruloplasmin is the higher concentration of calcium in drinking water and use of cooking oil, which contains the higher values of vitamin E. So, the main cause of heart disease in this table is the cooking oil and higher content of calcium bicarbonate in drinking water. If both causes, i.e., cooking oil and calcium in water are removed, the natural system can also recover the patients without using medicine.

### TABLE No. VI

The table shows the concentration of copper in the molecule of ceruloplasmin in normal female subjects without using cooking oil and iodized table salt in Kohat city of Pakistan. On comparison of the groups in serial No. 1 and 2, drinking water source is the same but the concentration of copper (due to the lesser number of the molecule of ceruloplasmin), is different which is attributed to the difference in eating habits. In the group of serial No. 1, the concentration of copper is higher due to the higher concentration of the molecule of ceruloplasmin as compared to the group of serial No. 2, which is due to the use of pulses in their food. So, the condition can be improved by avoiding the use of pulses in their food. On comparison of the groups in serial No. 3 and 4, the eating habits are the same but the concentration of copper is different which is due to the difference in calcium concentration of drinking water. The calcium content in drinking water of the group in serial No. 4 is high which lowers the concentration of the molecule of ceruloplasmin working as immune system against the heart disease. So, the groups of serial No. 2 and 4 are more prone to heart diseases.

### TABLE No. VII

The table shows the record of normal female subjects using cooking oil in Kohat city of Pakistan. All the groups in the table have lower values of copper in the molecule of ceruloplasmin irrespective of their eating habits and drinking water source. So, all the groups are more prone to heart diseases. The reason for the low value of copper in the molecule of ceruloplasmin is the use of cooking oil. By avoiding the use of cooking oil in food, the risk for the diseases can be reduced.

### TABLE No. VIII

The table shows the concentration of copper in the molecule of ceruloplasmin in female heart patients using cooking oil in Kohat city of Pakistan. The concentration of copper is low due to lesser number of the molecules of ceruloplasmin irrespective of their eating habits and drinking water source which is attributed to the use of cooking oil and is the major cause of disease.

### TABLE No. IX

The table shows the concentration of copper in the molecule of ceruloplasmin in normal male subjects without using cooking oil and iodized table salt in swat valley of Pakistan. All the groups have lower concentration of copper due to the low concentration of the molecule of ceruloplasmin irrespective of rinking water source. As all the groups use fish and pulses in their food which have high levels of vitamin E which is responsible for lowering of copper in the molecule of ceruloplasmin. So, all the groups are more prone towards the attack of heart diseases and by changing their eating habits, the risk for heart diseases can be reduced.

### TABLE No. X

The table shows the concentration of copper in the molecule of ceruloplasmin in normal male subjects without using cooking oil and iodized table salt in swat valley of Pakistan. All the groups in table have higher limits of copper n the molecule of ceruloplasmin as compared to the table number 1 X irrespective of the eating habits which may be attributed to the use of spring water. So, the deficiency of copper in the molecule of ceruloplasmin is overcome by the spring water which may have higher copper content and thus reduces the risk of heart diseases.

### TABLE No. XI

The table shows the concentration of copper in the molecule of ceruloplasmin in normal male subjects using cooking oil in swat valley of Pakistan. All the groups have low values of copper in the molecule of ceruloplasmin irrespective of their eating habits, which shows that all the groups using drinking water has higher calcium contents. Thus boiling the drinking water up to the limit that the maximum calcium is separated out can reduce the risk of heart diseases.

### TABLE No. XII

The table shows the copper content in the molecule of ceruloplasmin in male heart patients using cooking oil in swat valley of Pakistan. All the groups in the table have lower values of copper in the molecule of ceruloplasmin irrespective of their eating habits. The main cause for the lower values is the use of cooking oil and the disease can be overcome by avoiding the use of cooking oil even without the use of medicine.

### TABLE No. XIII

The table shows the values of copper in the molecule of ceruloplasmin in male heart patients without using cooking oil and allopathic medicine in swat (520) valley of Pakistan. The table shows higher values of copper in the molecule of ceruloplasmin irrespective of their eating habits, which shows that natural immune system due to the use of spring water tries to clean the blood vessels with pain.

### TABLE No. XIV

The table shows the values of copper in the molecule of ceruloplasmin in normal male subjects using cooking oil in Peshawar city of Pakistan. All the groups have lower concentration of copper in the molecule of ceruloplasmin irrespective of eating habits and drinking water source. The major reason for the lower concentration of copper in the molecule of ceruloplasmin is the use of cooking oil. So, the disease can be overcome by avoiding the use of cooking oil.

### TABLE No. XV

The table shows the concentration of copper in the molecule of ceruloplasmin in normal male subjects using iodized table salt in Peshawar city of Pakistan. All the groups have lower concentration of copper due to lower concentration of the molecule of ceruloplasmin irrespective of the eating habits and (535) drinking water source. So, all the groups are more prone to the heart diseases. Avoiding the use of iodized table salt can minimize the risk of heart disease.

### TABLE No. XVI

The table shows the concentration of copper in the molecule of ceruloplasmin in male heart patients using cooking oil in Peshawar city of Pakistan. All the groups have lower concentration of copper in the molecule of ceruloplasmin irrespective of the use of food and drinking water source. So, the main cause of the lower values of copper in the molecule of ceruloplasmin is cooking oil and the disease can be overcome by stopping the use of cooking oil.

## Claims

1. A solution of micronutrient copper sulphate for use in opening the blockage or narrowing of blood vessels in the treatment or prevention of heart diseases by producing ceruloplasmin.

2. The solution of claim 1, wherein said opening of the blockage or narrowing of blood vessels cures or prevents heart disease, except holes and low blood pressure, of a patient.

3. The solution of claim 1, wherein said opening of the blockage or narrowing of blood vessels cures or prevents high blood pressure of a patient.

4. A solution of micronutrient copper sulphate for use in curing brain hemorrhage of a patient by producing ceruloplasmin.

## Patentansprüche

1. Lösung des Mikronährstoffs Kupfersulfat zur Verwendung beim Öffnen des Verschlusses oder der Einengung von Blutgefäßen bei der Behandlung oder Abwendung von Herzkrankheiten durch Bilden von Ceruloplasmin.

2. Lösung nach Anspruch 1, wobei das Öffnen des Verschlusses oder der Einengung von Blutgefäßen Herzkrankheiten eines Patienten heilt oder abwendet, ausgenommen Löcher und Niedrigblutdruck.

3. Lösung nach Anspruch 1, wobei das Öffnen des Verschlusses oder der Einengung der Blutgefäße Bluthochdruck eines Patienten heilt oder abwendet.

4. Lösung des Mikronährstoffs Kupfersulfat zur Verwendung beim Heilen einer Hirnblutung eines Patienten durch Bilden von Ceruloplasmin.

## Revendications

1. Une solution de sulfate de cuivre micronutritive pour l'utilisation en l'ouvrage de l'obstruction ou du serrage des vaisseaux sanguine destinée au traitement ou à la prévention des maladie du coeur par la production de ceruloplasmine.

2. La solution de la revendication 1, dans laquelle dite ouvrage de l'obstruction ou du serrage des vaisseaux sanguine guérit ou prévient maladies de coeur, à l'exclusion de trous et hypotension artérielle, d'un patient.

3. La solution de la revendication 1, dans laquelle dite ouvrage de l'obstruction ou du serrage des vaisseaux sanguine guérit ou prévient hypertension artérielle d'un patient.

4. Une solution de cuivre micronutritive pour l'utilisation en guérir hémorragie cérébrale d'un patient par la production de ceruloplasmine.
